Europäisches Patentamt

European Patent Office

Office européen des brevets

(19)

(11) **EP 0 811 173 B1**

(12) # FASCICULE DE BREVET EUROPEEN

(45) Date de publication et mention
de la délivrance du brevet:
**02.05.2003 Bulletin 2003/18**

(51) Int Cl.⁷: **G02B 5/20**, A61F 9/00

(21) Numéro de dépôt: **96943165.9**

(22) Date de dépôt: **20.12.1996**

(86) Numéro de dépôt international:
**PCT/FR96/02049**

(87) Numéro de publication internationale:
**WO 97/023789 (03.07.1997 Gazette 1997/29)**

(54) **REJECTEUR SPECTRAL GRAND CHAMP ANGULAIRE ET PROCEDE DE FABRICATION**

WEITWINKLIGE SPEKTRALE UNTERDRÜCKUNGSVORRICHTUNG UND VERFAHREN ZU
IHRER HERSTELLUNG

WIDE-ANGLE SPECTRAL REJECTOR AND METHOD FOR MAKING SAME

(84) Etats contractants désignés:
**DE GB**

(30) Priorité: **21.12.1995 FR 9515259**

(43) Date de publication de la demande:
**10.12.1997 Bulletin 1997/50**

(73) Titulaire: **Thales**
**75008 Paris (FR)**

(72) Inventeurs:
• **JOUBERT, Cécile**
**F-94117 Arcueil Cédex (FR)**
• **LOISEAUX, Brigitte**
**F-94117 Arcueil Cédex (FR)**
• **HUIGNARD, Jean-Pierre**
**F-94117 Arcueil Cédex (FR)**

• **PUECH, Claude**
**F-94117 Arcueil Cédex (FR)**

(74) Mandataire: **Brochard, Pascale**
**Thales Intellectual Property**
**31-33,avenue Aristide Briand**
**94117 Arcueil Cedex (FR)**

(56) Documents cités:
**EP-A- 0 557 162          WO-A-83/04317**
**US-A- 5 103 323**

• **APPLIED PHYSICS LETTERS, vol. 65, no. 9, 29
Août 1994, pages 1079-1081, XP000464528 V.
LEYVA ET AL.: "Narrow bandwith volume
holographic optical filter operating at the Kr
transition at 1547.82 nm"**

EP 0 811 173 B1

## Description

L'invention concerne un rejecteur spectral grand champ angulaire pour la protection de certains systèmes optiques ou des yeux d'utilisateurs contre l'agression à certaines longueurs d'ondes. Par exemple, un tel rejecteur est utilisable dans une salle d'exposition aux rayonnements UV pour protéger les yeux du patient contre ces rayonnements UV.

Le système de l'invention doit réaliser une bonne protection contre une ou plusieurs longueurs d'ondes déterminées $\lambda_o$, c'est-à-dire limiter la transmission à ces longueurs d'ondes, tout en permettant la transmission aux autres longueurs d'ondes et ce pour un ensemble d'angles d'incidence appelé champ angulaire du composant $\Delta\theta$. Par exemple, le système de l'invention monté sur des lunettes de vision, empêchera l'utilisateur de percevoir la ou les longueurs d'ondes nocives tout en lui assurant la vision normale du paysage environnant.

Les performances recherchées sont donc les suivantes :

- une réflectivité du composant $R(\lambda)$ très importante pour $\lambda_o$ mais la plus faible possible pour les longueurs d'onde voisines de $\lambda_o$.
- on définit la densité optique D par

$$T = 10^{-D}$$

  T transmission du composant
- densité optique Do du composant à la longueur d'onde $\lambda_o$ pour un champ angulaire $\Delta\theta$ :
  $D(\theta) \geq D_o$ pour $\theta$ compris dans la bande [$\theta_o$-$\Delta\theta$ ; $\theta_o$+$\Delta\theta$] pour $\lambda = \lambda_o$

On sait réaliser une telle fonction à l'aide d'un réseau de Bragg en réflexion. Un réseau de Bragg en réflexion est un matériau dont l'indice de réfraction est modulé sinusoïdalement dans l'épaisseur avec une période $\Lambda$. Ce réseau réfléchit la longueur d'onde $\lambda_o$ d'un faisceau incident selon la direction normale au support du matériau si son pas $\Lambda$ vérifie $\Lambda=\lambda_o/2n$ n indice moyen du matériau (condition de Bragg). Par exemple, pour se protéger d'un faisceau à la longueur d'onde $\lambda_o = 532$ nm émis par un laser YAG doublé, on prévoit de mettre sur chaque verre d'une paire de lunette de vision, une couche d'un matériau formant un réseau de Bragg tel que le faisceau de longueur d'onde $\lambda_o$ à incidence normale sur le plan du réseau soit réfléchi.

Dans le cas d'un réseau épais de type Bragg, les variations de la densité optique D en fonction de l'angle d'incidence $\theta$ et de la longueur d'onde incidente $\lambda$ sont principalement fonction de l'épaisseur d du matériau et de sa modulation d'indice $\Delta n$.

D'une façon générale, pour un composant fonctionnant en réflexion :

- le point de fonctionnement du matériau, exprimé sous la forme du couple ($\Delta n$-modulation d'indice, d-épaisseur du matériau), permettant d'obtenir une densité donnée $D_o$ à $\lambda_o$ et pour une incidence donnée $\theta_o$ est du type $\Delta n.d$ = cste.
  Pour avoir une transmission du composant maximale pour les longueurs d'ondes voisines de $\lambda_o$ (réflectivité $R(\lambda)$ en fonction de la longueur d'onde étroite), on a intérêt à se placer à un point de fonctionnement correspondant à l'épaisseur d la plus grande possible, et donc une modulation d'indice $\Delta n$ la plus faible possible.
- le champ angulaire $\Delta\theta$ au voisinage de l'incidence moyenne $\theta_o$, correspondant à la protection laser recherchée ($D(\theta) \geq D_o$) est proportionnel à $\Delta n$. La bande passante $\Delta\lambda$ de la réflectivité spectrale $R(\lambda)$ pour $\theta = \theta_o$, varie également de la même manière. Il est donc contradictoire de rechercher un réseau de Bragg avec champ angulaire $\Delta\theta$ important et une bande passante $\Delta\lambda$ étroite.

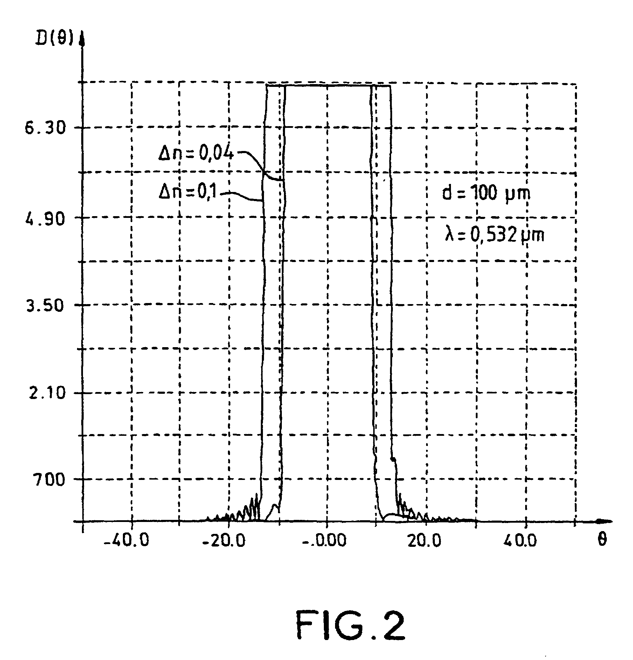

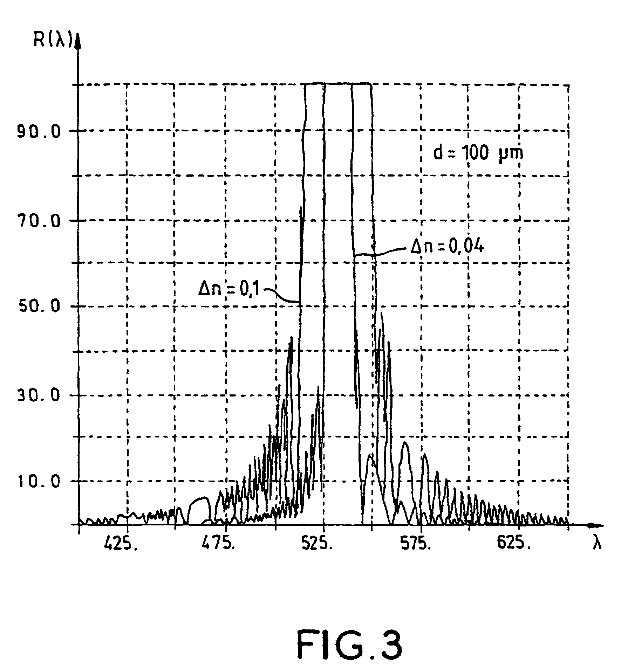

A titre d'exemple, on donne en figure 2 pour d = 100 µm les courbes $D(\theta)$ de densité optique D en fonction de l'angle d'incidence $\theta$ où $\lambda = \lambda_o = 0,532$ µm et pour des modulation d'indices $\Delta n = 0,1$ et $\Delta n = 0,04$. La figure 3 donne également pour d = 100 µm, et des modulations d'indices $\Delta n = 0,1$ et $\Delta n = 0,04$ les courbes à incidence nulle ($\theta = \theta_o = 0°$), $R(\lambda)$ de réflectivité en fonction de la longueur d'onde autour de la longueur d'onde centrale (0,532 µm). La courbe $D(\theta)$ plafonne à D = 7 pour une meilleure lisibilité de la courbe. Les angles d'incidence $\theta$ sont donnés dans le milieu d'indice n = 1,5.

On résume ci-dessous les valeurs du champ angulaire $\Delta\theta$ tel que $D(\theta) \geq 4$ pour deux points de fonctionnement ainsi que la bande passante $\Delta\lambda$ de la réflectivité spectrale.

(100 µm.$\Delta n$ = 0.04) : $\Delta\theta = \pm 14°$ air      $\Delta\lambda = 15$ nm

(100 µm.$\Delta n$ = 0.08) : $\Delta\theta = \pm 20°$ air      $\Delta\lambda = 40$ nm

Le point de fonctionnement (100 µm.$\Delta n$ = 0.08) satisfait bien la condition de champ angulaire ($\Delta\theta \geq \pm 20°$) mais sa réflectivité spectrale $\Delta\lambda$ est beaucoup trop élevée, elle perturberait considérablement la vision des couleurs dont les longueurs d'ondes sont situées de part et d'autre de la longueur d'onde à éliminer. Au travers de cet exemple, on a montré qu'il n'y a donc pas de solution satisfaisante avec une configuration à un miroir de Bragg classique lorsque le champ angulaire demandé est élevé.

L'invention permet de résoudre ce problème en fournissant un dispositif qui permet de rejeter la lumière d'une longueur déterminée qui est incidente sur le dispositif selon un grand champ angulaire et ce avec une réflectivité $R(\lambda)$ plus étroite que dans les dispositifs connus.

Des dispositifs comprenant un empilement d'hologrammes permettant de filtrer des rayons lumineux sur une grande plage angulaire sont décrits dans les documents US 5 103 323 et EP 0 557 162.

L'invention concerne, selon la revendication 1, un rejecteur spectral comprenant un support transparent à une large gamme de longueurs d'ondes muni d'une couche d'un matériau comportant un premier réseau de Bragg réflecteur à une longueur d'onde déterminée, dont les strates sont inclinées selon une première inclinaison par rapport au support du réseau, caractérisé en ce que ladite couche de matériau comporte au moins un deuxième réseau de Bragg dont les strates ont une deuxième inclinaison différente de celle du premier réseau.

L'invention concerne également, selon la revendication 10, un procédé de fabrication d'un rejecteur spectral qui prévoit de faire interférer dans une même couche de matériau polymère successivement plusieurs faisceaux optiques d'incidences différentes ; les réseaux sont ainsi multiplexés dans une même couche.

Un autre procédé, selon la revendication 12, prévoit de faire interférer dans une même couche de matériau photosensible, un faisceau de rayons parallèles avec un faisceau de rayons issus d'un diffuseur.

Les différents objets et caractéristiques de l'invention apparaîtront plus clairement dans la description qui va suivre et dans les figures annexées qui représentent :

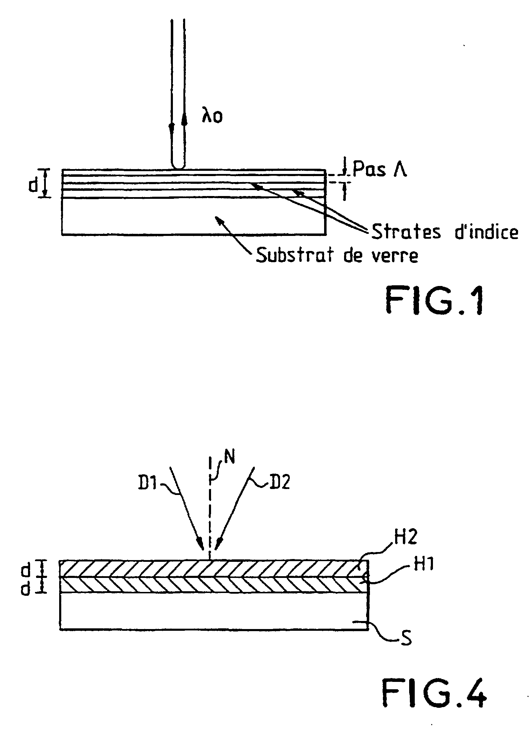

- la figure 1, un dispositif à réseau de Bragg connu dans la technique ;
- les figures 2 et 3, des courbes de fonctionnement du dispositif de la figure 1 ;
- la figure 4, un exemple de dispositif rejecteur grand angle selon l'invention ;
- les figures 5a et 5b, un exemple de réalisation du dispositif rejecteur de la figure 4 ;
- les figures 6a, 6b et 7, le fonctionnement du rejecteur des figures 5a et 5b ;
- les figures 8 à 10, des courbes de fonctionnement ;
- les figures 11a et 11b, une variante de réalisation selon l'invention.

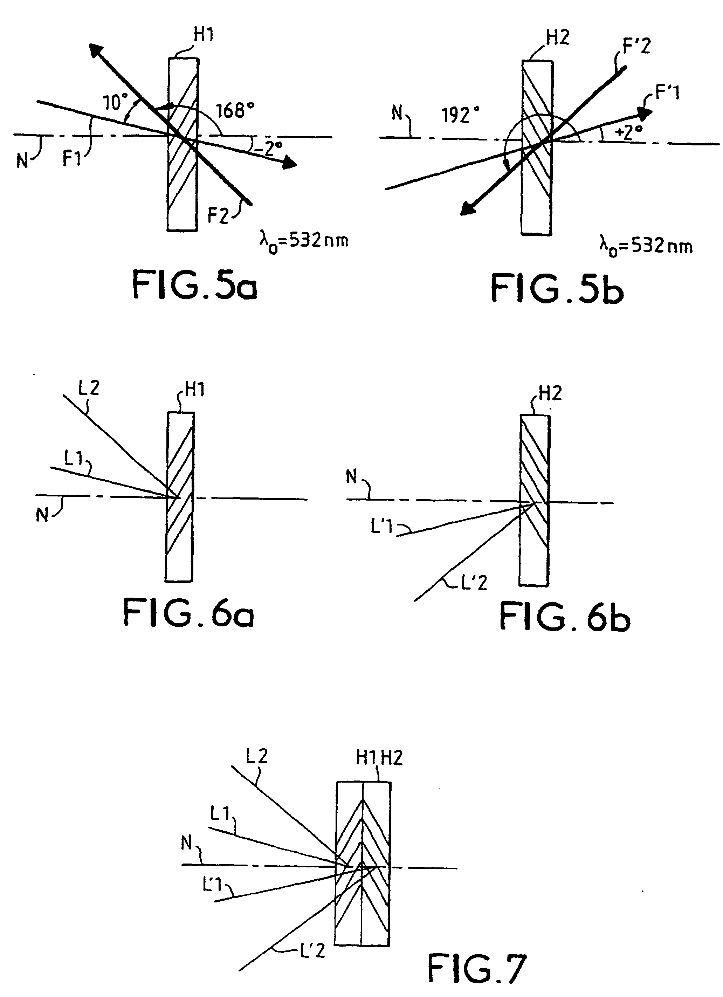

L'invention consiste à combiner au moins deux composants holographiques dont les strates d'indice sont inclinées par rapport à la direction d'incidence moyenne de la lumière. La figure 4 représente un tel rejecteur.

Il comporte sur un substrat S deux réseaux holographiques H1, H2 dont les strates d'indice sont inclinées par rapport au plan du substrat. De façon préférentielle, mais cela n'est pas obligatoire, les inclinaisons des strates d'un réseau sont symétriques de celles de l'autre réseau par rapport à la normale au plan du substrat. Les réseaux sont fabriqués pour être réflecteur à une longueur d'onde.

Le substrat est transparent à une large gamme de longueurs d'ondes et de façon courante cette gamme de longueurs d'ondes englobe toutes les longueurs d'ondes du visible.

De façon générale, les deux réseaux sont conçus de façon à réfléchir la lumière incidente globalement selon des directions D1, D2 symétriques par rapport à la normale N au plan du substrat.

Pour mieux comprendre le fonctionnement détaillé de ces réseaux on va décrire leur réalisation en se reportant aux figures 5a et 5b. Selon ces figures, les deux réseaux sont enregistrés dans le même film, dans le mode de réalisation principal de l'invention, soit séquentiellement, soit simultanément.

Chaque réseau H1 et H2 est enregistré par deux ondes planes non symétriques par rapport à la normale au plan du film photosensible. Les réseaux d'indice obtenus sont alors inclinés par rapport au support. Pour un réseau de Bragg enregistré avec deux faisceaux F1, F2 à la longueur d'onde $\lambda_o$, la réflectivité du composant est maximale à $\lambda_o$ pour les deux directions de faisceaux F1 F2. En choisissant correctement les angles d'enregistrement des deux réseaux H1 et H2, il est possible de juxtaposer leur densité angulaire $D(\theta)$, de façon à agrandir la densité $D(\theta)$ de l'ensemble, sans augmenter $\Delta\lambda$. La réalisation de ces composants s'effectue de manière aisée dans des photopolymères ayant une modulation d'indice maximale de $\Delta n = 0.06$ et une épaisseur maximale d'environ 50 $\mu m$. En effet, contrairement à la gélatine bichromatée dont le traitement humide rend plus délicate l'enregistrement de strates inclinées, ceux-ci ne nécessitent pas de contraintes particulières pour maintenir la direction inclinée des strates d'indices.

A titre d'exemple, le réseau H1 est enregistré (voir figure 5a) par l'interférence dans le milieu photosensible d'un premier faisceau F1 (à une longueur d'onde $\lambda_o$) faisant un angle de 2° avec la normale N et d'un deuxième faisceau F2 (à la même longueur d'onde $\lambda_o$) faisant un angle de 10° avec le premier faisceau, ces deux faisceaux se propagent en sens inverse mais du même côté par rapport à la normale N.

Le réseau H2 est enregistré (figure 5b) à l'aide de deux faisceaux F'1 et F'2 (de longueurs d'ondes $\lambda_o$) symétriques respectivement des faisceaux F1 et F2 par rapport à la normale N. Cette symétrie n'est pas obligatoire mais peut être avantageuse.

L'utilisation de ces réseaux se fait de la manière suivante. Le réseau H1 présente deux maxima de réflexion pour les directions L1 et L2 (figure 6a). De même, pour le réseau H2 (figure 6b) la réflexion est maximum pour la lumière incidente selon l'une ou l'autre des directions L'1, L'2. Les directions L1, L2, L'1. L'2 correspondent aux directions des faisceaux d'enregistrement F1, F2, F'1, F'2 respectivement. Bien entendu, ces réseaux fonctionnent à la même longueur d'onde $\lambda_o$ que celle des faisceaux d'enregistrement.

Avec les deux films H1 et H2 ainsi enregistrés comme cela est représenté en figure 7, on obtient un dispositif présentant quatre directions d'incidence de la lumière pour lesquelles la réflexion est maximale à la

longueur d'onde $\lambda_o$.

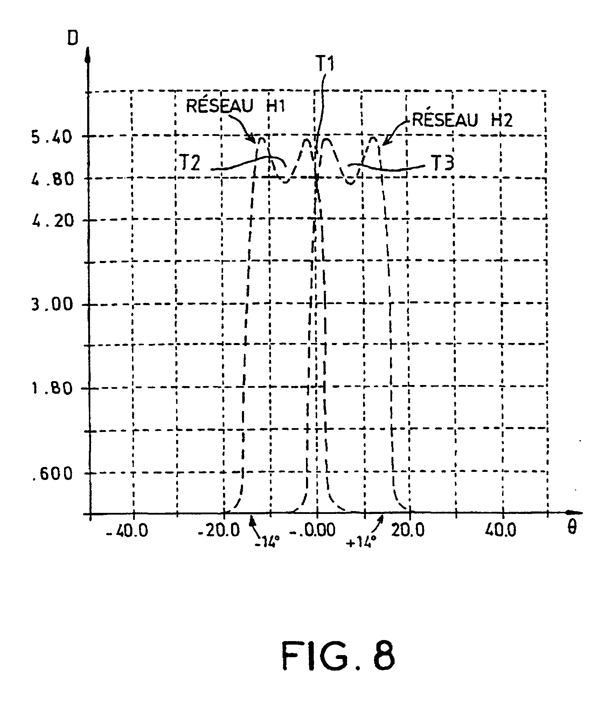

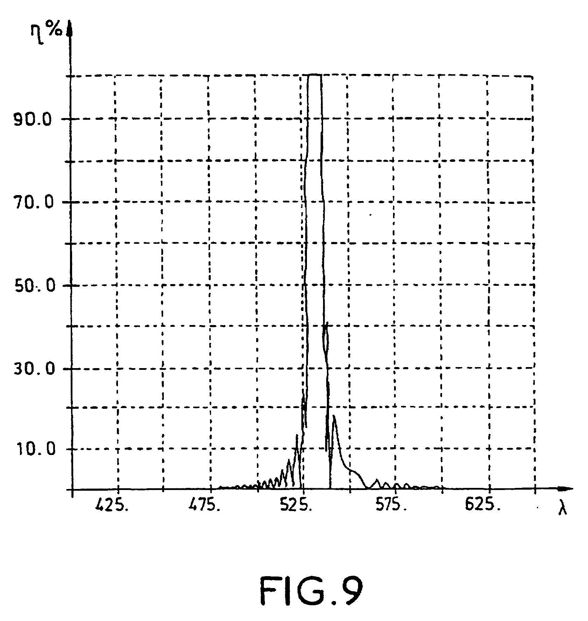

Les deux courbes D(θ) de chaque composant du rejecteur sont décrites sur la figure 8 pour des réseaux ayant d = 50 μm Δn = 0.023. On voit que la densité optique est supérieure à 4 sur un champ angulaire Δθ = ± 13° milieu = ± 20° air. La réflectivité R(λ) correspondant à un des réseaux est donnée figure 9. L'angle d'incidence correspond à une incidence de Bragg par exemple θ = -2° dans le milieu (onde plane). La bande passante spectrale n'est ici que de 10 nm, à comparer avec les 40 nm pour la configuration classique et dans les mêmes conditions de lecture, Δn est plus faible (0,023 < 0,06) que dans la configuration classique.

En effet, puisque l'on peut augmenter la densité angulaire D(9) par juxtaposition de deux réseaux inclinés à faible Δn, la bande passante angulaire initiale de chaque réseau est bien inférieure au champ angulaire spécifié, par exemple Δθ = ± 20°. Cette levée de contrainte sur la densité angulaire permet d'enregistrer les réseaux élémentaires H1 ou H2 avec un point de fonctionnement comportant une modulation d'indice Δn plus faible que dans le cas classique, et à une modulation d'indice plus faible correspond une réflectivité spectrale R(λ) plus étroite.

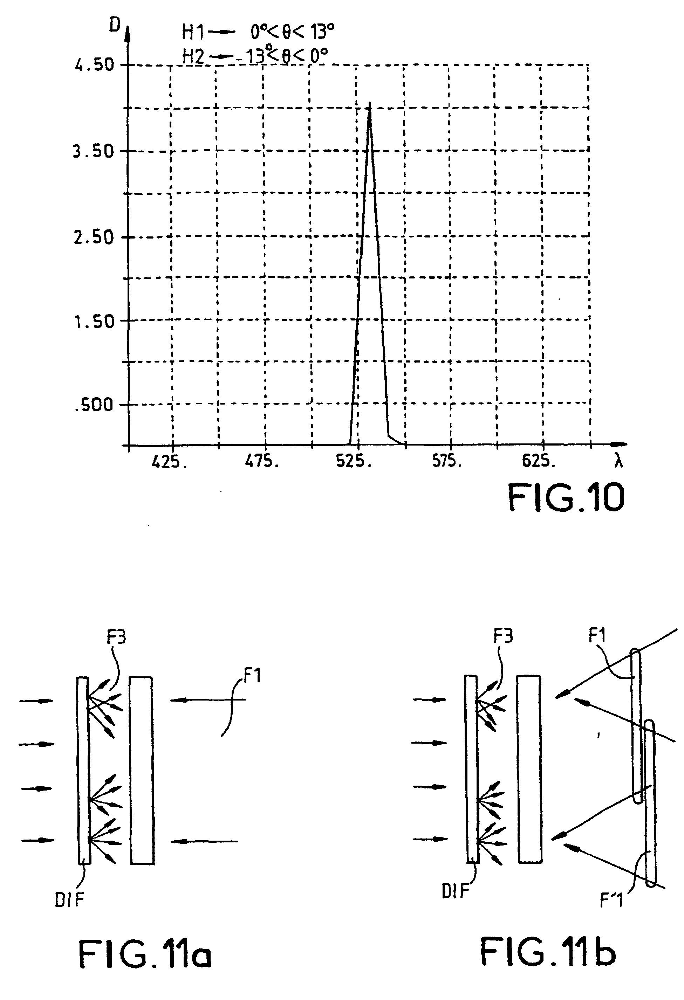

En pratique, la lumière éclaire le composant selon un champ angulaire Δθ (jumelle, épiscope, intensificateur d'image ...). Il convient d'intégrer la réflectivité R(λ) sur tous les angles d'incidence potentiels. La courbe résultante en juxtaposant les deux réseaux H1 et H2 est donnée figure 10 pour un champ angulaire Δθ = ± 20° air. D(λ) correspond donc à la densité optique du composé rejecteur en fonction de la longueur d'onde, intégrée sur l'ensemble du champ angulé Δθ = ± 20° air.

On voit que pour λ = $\lambda_o$, D(λ) ≥ 4 sur l'ensemble du champ angulaire, ce qui est le résultat souhaité. D'autre part, la densité optique chute ensuite très vite autour de $\lambda_o$. La bande passante Δλ rejectée par le composant est de Δλ =20 nm.

L'utilisation de deux réseaux H1, H2 dont les strates sont inclinées de façon complémentaire permet ainsi de garantir une réflexion avec une densité optique donnée sur un grand champ angulaire. Cependant, ce système fonctionne dans le plan d'incidence, c'est-à-dire le plan contenant les deux faisceaux (F1, F2 d'une part et F'1, F'2 d'autre part) utilisés lors de l'enregistrement des réseaux. Pour couvrir l'ensemble de l'espace, il convient d'associer deux réseaux identiques aux réseaux H1, H2 mais tournés de π/2 autour de la normale au support.

Selon une variante, on peut également, pour augmenter le champ angulaire, associer plus de deux réseaux.

Par exemple, on peut prévoir tout d'abord un réseau fonctionnant à incidence normale (strates parallèles au plan du support) de façon à rejecter le champ angulaire Δθ autour de la normale. On peut aussi associer d'autres réseaux enregistrés avec des faisceaux inclinés de façon à rejecter $\lambda_o$ sur le champ angulaire de part et d'autre de la normale. On peut également prévoir des réseaux dont les courbes de fonctionnement sont situées de part et d'autre des courbes de la figure 8.

Selon une autre variante, on peut associer plusieurs réseaux enregistrés avec des longueurs d'ondes différentes et réfléchissant ces longueurs d'ondes. Le rejecteur assurera donc une protection contre plusieurs longueurs d'ondes.

La réalisation pratique d'un tel rejecteur spectral peut être obtenue par juxtaposition de réseaux H1 et H2.

Il est également possible dans une même couche de matériau photosensible, de réaliser une première interférence lumineuse (avec les faisceaux F1, F2) puis une deuxième interférence lumineuse (avec les faisceaux F'1, F'2). Cela est possible en tenant compte du fait que la somme des différences d'indice Δn et Δ'n créées par les deux interférences doit être inférieure à la variation d'indice maximum que l'on peut induire dans la couche photosensible. On obtient ainsi un multiplexage de réseaux.

Une variante de rejecteur consiste comme cela est illustré par la figure 11a, à faire interférer, dans le matériau photosensible H, un premier faisceau F2 d'une direction déterminée (par exemple perpendiculaire au plan de la couche de matériau photosensible) et un deuxième faisceau divergent F3 obtenu par transmission de ce faisceau au travers d'un diffuseur DIF par exemple. Le faisceau F3 comporte un ensemble de faisceaux d'angles différents. Une multitude de réseaux correspondant aux différents angles est enregistrée dans le matériau.

Le champ angulaire du composant rejecteur est donc augmenté.

De plus, on peut envisager d'utiliser la méthode des deux réseaux inclinés décrite plus haut non plus avec, pour l'enregistrement de deux faisceaux type onde plane, mais avec un des faisceaux type diffuseur. Cette variante est représentée par la figure 11b où l'on prévoit l'interférence d'un faisceau diffusant F3 avec d'une part un faisceau F1 incliné selon une certaine incidence et, d'autre part, un faisceau F'1 présentant une autre incidence.

Dans ce qui précède, pour faciliter les explications, on a considéré que les longueurs d'ondes utilisées pour l'enregistrement des réseaux étaient celles de la longueur d'onde à rejecter. En réalité, les longueurs d'ondes d'enregistrement peuvent être différentes de celles de l'utilisation. Dans ce cas, les directions pour lesquelles les rejections seront maximales, ne correspondront pas aux directions des ondes d'enregistrement.

**Revendications**

1. Rejecteur spectral comprenant un support transparent à une large gamme de longueurs d'ondes muni d'une couche d'un matériau comportant un premier réseau de Bragg (H1) réflecteur à une longueur d'onde déterminée, dont les strates (H1) sont inclinées selon une première inclinaison par rapport au support du réseau, **caractérisé en ce que** ladite couche de matériau comporte au moins un deuxième réseau (H$_2$) de Bragg dont les strates ont une deuxième inclinaison différente de celle du premier réseau.

2. Rejecteur spectral selon la revendication 1, **caractérisé en ce que** les première et deuxième inclinaisons sont symétriques par rapport à la normale.

3. Rejecteur spectral selon la revendication 1, **caractérisé en ce que** les premier et deuxième réseaux (H1, H2) comportent chacun deux directions d'incidence (L1, L2) selon lesquelles les réflexions sont maximales.

4. Rejecteur spectral selon l'une des revendications 2 ou 3, **caractérisé en ce que** les directions d'incidence pour lesquelles les réflexions sont maximales pour un réseau (H1) sont symétriques de celles de l'autre réseau (H$_2$) par rapport à la normale au plan des réseaux.

5. Rejecteur spectral selon la revendication 1, **caractérisé en ce qu'**il comporte un troisième réseau dont les strates sont parallèles au support.

6. Rejecteur spectral selon la revendication 1, **caractérisé en ce qu'**il comporte plus de deux réseaux, les différents réseaux présentant des strates inclinées différemment d'un réseau à l'autre.

7. Rejecteur spectral selon la revendication 5 ou 6, **caractérisé en ce que** les différents réseaux sont inscrits dans des couches de matériaux juxtaposés.

8. Rejecteur spectral selon la revendication 5 ou 6, **caractérisé en ce que** les différents réseaux sont inscrits dans une même couche de matériau photosensible.

9. Rejecteur spectral selon la revendication 1, **caractérisé en ce qu'**il comporte une pluralité de réseaux permettant la réflexion diffuse d'une onde plane incidente.

10. Procédé de fabrication d'un rejecteur spectral selon la revendication 1, **caractérisé en ce qu'**il prévoit de faire interférer dans une même couche de matériau photosensible successivement plusieurs faisceaux optiques d'incidences différentes.

11. Procédé selon la revendication 10, **caractérisé en ce que** chaque réseau est inscrit dans une couche de matériau photosensible par interférence de deux faisceaux dont les directions correspondent aux directions d'incidences pour lesquelles le réseau doit présenter des taux de réflexion maximum.

12. Procédé de fabrication d'un rejecteur spectral selon la revendication 1, **caractérisé en ce qu'**il prévoit de faire interférer, dans une même couche de matériau photosensible, un premier faisceau de rayons parallèles (F2) avec un faisceau de rayons (F3) issu d'un diffuseur (DIF).

13. Procédé de fabrication d'un rejecteur spectral selon la revendication 12, **caractérisé en ce que** le premier faisceau de rayon parallèle est incliné par rapport à la normale au plan du support, et qu'il prévoit de faire interférer, dans une même couche de matériau photosensible, le faisceau de rayons issus du diffuseur avec un deuxième faisceau de rayons parallèles inclinés par rapport à la normale au plan du support différemment de l'inclinaison du premier faisceau de rayons parallèles.

**Patentansprüche**

1. Spektrale Sperre, mit einem Träger, der in einem großen Wellenlängenbereich lichtdurchlässig ist und mit einer Materialschicht versehen ist, die ein erstes Bragg-Gitter (H1), das bei einer bestimmten Wellenlänge reflektiert und dessen Lagen (H1) in einer ersten Neigungsrichtung in bezug auf den Träger des Gitters geneigt sind, umfaßt, **dadurch gekennzeichnet, daß** die Materialschicht wenigstens ein zweites Bragg-Gitter (H2) umfaßt, dessen Lagen eine zweite Neigungsrichtung besitzen, die von jener des ersten Gitters verschieden ist.

2. Spektrale Sperre nach Anspruch 1, **dadurch gekennzeichnet, daß** die erste und die zweite Neigungsrichtung in bezug auf die Normale symmetrisch sind.

3. Spektrale Sperre nach Anspruch 1, **dadurch gekennzeichnet, daß** das erste Gitter und das zweite Gitter (H1, H2) jeweils zwei Einfallsrichtungen (L1, L2) aufweisen, längs derer die Reflexionen maximal sind.

4. Spektrale Sperre nach einem der Ansprüche 2 oder 3, **dadurch gekennzeichnet, daß** die Einfallsrichtungen, für die die Reflexionen für ein Gitter (H1) maximal sind, zu jenen des anderen Gitters (H2) in bezug auf die Normale der Ebene der Gitter symmetrisch sind.

5. Spektrale Sperre nach Anspruch 1, **dadurch gekennzeichnet, daß** sie ein drittes Gitter aufweist, dessen Lagen zum Träger parallel sind.

**6.** Spektrale Sperre nach Anspruch 1, **dadurch gekennzeichnet, daß** sie mehr als zwei Gitter umfaßt, wobei die verschiedenen Gitter Lagen aufweisen, die von einem Gitter zum nächsten unterschiedlich geneigt sind.

**7.** Spektrale Sperre nach Anspruch 5 oder 6, **dadurch gekennzeichnet, daß** die verschiedenen Gitter in nebeneinander angeordnete Materialschichten eingebracht sind.

**8.** Spektrale Sperre nach Anspruch 5 oder 6, **dadurch gekennzeichnet, daß** die verschiedenen Gitter in dieselbe Schicht aus einem lichtempfindlichen Material eingebracht sind.

**9.** Spektrale Sperre nach Anspruch 1, **dadurch gekennzeichnet, daß** sie mehrere Gitter aufweist, die die diffuse Reflexion einer einfallenden ebenen Welle ermöglichen.

**10.** Verfahren für die Herstellung einer spektralen Sperre nach Anspruch 1, **dadurch gekennzeichnet, daß** es vorsieht, in derselben Schicht aus einem lichtempfindlichen Material nacheinander mehrere Lichtstrahlenbündel mit unterschiedlichen Einfallswinkeln zur Interferenz zu bringen.

**11.** Verfahren nach Anspruch 10, **dadurch gekennzeichnet, daß** jedes Gitter in eine Schicht aus einem lichtempfindlichen Material durch Interferenz zweier Strahlenbündel eingebracht wird, deren Richtungen jenen Einfallsrichtungen entsprechen, für die das Gitter maximale Reflexionsbeträge aufweisen soll.

**12.** Verfahren für die Herstellung einer spektralen Sperre nach Anspruch 1, **dadurch gekennzeichnet, daß** es vorsieht, in derselben Schicht aus einem lichtempfindlichen Material ein erstes Strahlenbündel (F2) aus parallelen Strahlen mit einem Strahlenbündel (F3), das von einem Diffusor (DIF) stammt, zur Interferenz zu bringen.

**13.** Verfahren für die Herstellung einer spektralen Sperre nach Anspruch 12, **dadurch gekennzeichnet, daß** das erste parallele Strahlenbündel in bezug auf die Normale der Trägerebene geneigt ist und daß es vorsieht, in derselben Schicht aus einem lichtempfindlichen Material das Strahlenbündel aus vom Diffusor stammenden Strahlen mit einem zweiten Strahlenbündel aus parallelen Strahlen, die in bezug auf die Normale der Trägerebene anders als das erste Strahlenbündel aus parallelen Strahlen geneigt sind, zur Interferenz zu bringen.

## Claims

**1.** Spectral rejector comprising a substrate transparent over a wide range of wavelengths and provided with a layer of a material that includes a first Bragg grating (H1) reflecting at a defined wavelength, the strata (H1) of which are inclined at a first angle of inclination to the substrate of the grating, **characterized in that** the said layer of material includes at least a second Bragg grating (H2), the strata of which have a second angle of inclination different from that of the first grating.

**2.** Spectral rejector according to Claim 1, **characterized in that** the first and second angles of inclination are symmetrical with respect to the normal.

**3.** Spectral rejector according to Claim 1, **characterized in that** the first and second gratings (H1, H2) each have two directions of incidence (L1, L2) along which the reflections are at a maximum.

**4.** Spectral rejector according to either of Claims 2 and 3, **characterized in that** the directions of incidence for which the reflections are at maximum for a grating (H1) are symmetrical with those of the other grating (H2) with respect to the normal of the plane of the gratings.

**5.** Spectral rejector according to Claim 1, **characterized in that** it includes a third grating whose strata are parallel to the substrate.

**6.** Spectral rejector according to Claim 1, **characterized in that** it includes more than two gratings, the various gratings having strata inclined differently from one grating to another.

**7.** Spectral rejector according to Claim 5 or 6, **characterized in that** the various gratings are written into layers of juxtaposed materials.

**8.** Spectral rejector according to Claim 5 or 6, **characterized in that** the various gratings are written into the same layer of photosensitive material.

**9.** Spectral rejector according to Claim 1, **characterized in that** it comprises a plurality of gratings allowing diffuse reflection of an incident plane wave.

**10.** Process for manufacturing a spectral rejector according to Claim 1, **characterized in that** it provides for several optical beams of different angles of incidence to be made to interfere in succession in the same layer of photosensitive material.

**11.** Process according to Claim 10, **characterized in that** each grating is written into a layer of photosensitive material by interference of two beams, the directions of which correspond to the directions of incidence for which the grating must have maximum levels of reflection.

**12.** Process for manufacturing a spectral rejector according to Claim 1, **characterized in that** it provides for a first beam of parallel rays (F2) to be made to interfere, in the same layer of photosensitive material, with a beam of rays (F3), coming from a diffuser (DIF).

**13.** Process for manufacturing a spectral rejector according to Claim 12, **characterized in that** the first beam of parallel rays is inclined to the normal to the plane of the substrate and **in that** provision is made for the beam of rays coming from the diffuser to be made to interfere, in the same layer of photosensitive material, with a second beam of parallel rays inclined to the normal to the plane of the substrate differently from the inclination of the first beam of parallel rays.

λo

d

Pas Λ

Strates d'indice

Substrat de verre

# FIG.1

N

D1

D2

d
d

H2

H1

S

# FIG.4

FIG.2

FIG.3

H1

10°    168°

N    F1    −2°

F2

$\lambda_0 = 532\,nm$

FIG.5a

H2    F'2

F'1

N    192°    +2°

$\lambda_0 = 532\,nm$

FIG.5b

L2    H1

L1

N

FIG.6a

H2

N

L'1

L'2

FIG.6b

L2    H1 H2

L1

N

L'1

L'2

FIG.7

FIG. 8

FIG.9

FIG.10

FIG.11a

FIG.11b